# EUROPÄISCHE PATENTSCHRIFT

(11) **EP 1 055 122 B1**
(45) Veröffentlichungstag und Bekanntmachung des Hinweises auf die Patenterteilung: **11.12.2002**
(21) Anmeldenummer: 99904826.7
(22) Anmeldetag: 29.01.1999
(51) Int. Cl.: G01N 33/00

(54) **BAUELEMENT ZUR GASKONZENTRATIONSMESSUNG**
COMPONENT FOR MEASURING GAS CONCENTRATIONS
COMPOSANT POUR MESURER LA CONCENTRATION DE GAZ

(30) Priorität: 11.02.1998 AT 7498 U
(43) Veröffentlichungstag der Anmeldung: 29.11.2000
(73) Patentinhaber: E + E ELEKTRONIK GESELLSCHAFT M.B.H., A-4210 Engerwitzdorf (AT)
(72) Erfinder: WAGNER, Dieter, A-4040 Linz (AT); HARTL, Josef, A-4040 Linz (AT)
(74) Vertreter: Hofmann, Ernst
(86) Internationale Anmeldenummer: EP9900585
(87) Internationale Veröffentlichungsnummer: WO99041603

(56) Entgegenhaltungen:
- EP-A- 0 571 115
- EP-A- 0 674 171
- WO-A-91/03735
- WO-A-97/13147
- PATENT ABSTRACTS OF JAPAN vol. 7, no. 90 (P-191), 14. April 1983 & JP 58 015147 A (KOBAYASHI TOYOHIRO), 28. Januar 1983
- PATENT ABSTRACTS OF JAPAN vol. 006, no. 209 (P-150), 21. Oktober 1982 & JP 57 116242 A (HITACHI SEISAKUSHO KK), 20. Juli 1982

## Beschreibung

Die vorliegende Erfindung betrifft ein Bauelement zur Gaskonzentrationsmessung, welches insbesondere zur automatisierten Bestückung von Leiterplatten, Platinen oder dgl. geeignet ist.

Zur Gaskonzentrationsmessung existieren verschiedenste Sensortypen, beispielsweise Feuchtesensoren, mit deren Hilfe z.B. die Konzentration von Wasserdampf in Luft bestimmt wird. In der Klimatierungstechnik eingesetzte Feuchtesensoren bestehen in der Regel aus einem Gehäuse, in dem das eigentliche feuchteempfindliche Sensorelement angeordnet ist. Oft ist hierbei desweiteren auch der Einbau eines Filtermateriales vor der feuchteempfindlichen Fläche des Sensorelementes vorgesehen, welches einerseits durchlässig für die umgebende Feuchte ist und andererseits einen mechanischen Schutz vor Verunreinigungen für das Sensorelement bildet.
Eine übliche Bauform sieht etwa vor, das Sensorelement in einem Fühlerkopf anzuordnen, wobei das Sensorelement durch einen für die jeweilige Applikation geeigneten Filter umgeben ist. Derartige Feuchtesensoren sind etwa aus der Firmendruckschrift SENSOR TECHNOLOGY" der Anmelderin unter der Typenbezeichnung Serie EE 06 bekannt.
Im Fall des Einsatzes in der Klimatisierungstechnik werden derartig montierte Feuchtesensoren meist mit einer Auswerteelektronik versehen und das komplette Bauteil unter dem Begriff Transmitter" eingesetzt. Hierbei bereitet es in der Regel keine Probleme, ein derartiges zylinderförmiges Bauelement an einer geeigneten Stelle anzuordnen. In anderen Anwendungen solcher Bauelemente, z.B. im Bereich der KFZ-Elektronik. kann es nunmehr jedoch erforderlich sein, ein derartiges Bauelement zur Feuchtemessung auf einer Leiterplatte, auf einer Platine oder dgl. anzuordnen und geeignet zu kontaktieren. Grundsätzlich wäre dies zwar auch mit dem oben erwähnten Bauelement möglich; eine automatisierte Bestückung mittels bekannter Leiterplatten-Bestückungsmethoden, z.B. in Form der SMD-Bestückung, ist mit einem solchermaßen aufgebauten Bauelement jedoch nicht möglich.

Ein Bauelement zur Gaskonzentrationsmessung in Form eines weiteren Feuchtesensorelementes, welches auf einer Leiterplatte angeordnet wird, ist numehr etwa aus der EP 0 571 115 A2 bekannt. Hierbei ist vorgesehen, das eigentliche Sensorelement steckbar auf der Leiterplatte anzuordnen. Weitere Details zur Kontaktierung bzw. zur konkreten Ausbildung des Sensorelementes enthält diese Druckschrift jedoch nicht.

Die WO-A-97 13147 offenbart einen gassensor mit einem teildurchlässigen Element, welches das zu detektierende gas durchläßt. Dieses Element ist in ein Basismaterial integriert, welches den Sensor umgibt. Insbesondere ist der Sensor in direktem Kontakt mit dem teildurchlässigen Element.

Die der vorliegenden Erfindung zugrundeliegende Aufgabenstellung wurde bislang primär am Beispiel eines als Feuchtsensors ausgebildeten Bauelementes zur Gaskonzentrationsmessung erläutert. Grundsätzlich identische Aufgabenstellungen ergeben sich jedoch auch bei anderen Bauelementen zur Gaskonzentrationsmessung, wie beispielsweise CO₂-Sensoren etc., mit denen etwa die CO₂-Konzentration in Luft gemessen werden soll.

Aufgabe der vorliegenden Erfindung ist es daher, ein Bauelement zur Gaskonzentrationsmessung zu schaffen, welches zur automatisierten Bestückung von Leiterplatten, Platinen oder dgl. geeignet ist. Desweiteren wird dabei ein möglichst einfacher Aufbau des Bauelementes in Verbindung mit der erforderlichen Stabilität gegenüber mechanischer Belastung gefordert. Darüberhinaus sollte selbstverständlich gewährleistet sein, daß die jeweiligen Sensorelemente mit dem zu bestimmenden Gas hinreichend in Kontakt kommen.

Diese Aufgabe wird gelöst durch ein Bauelement mit den Merkmalen des Anspruches 1 oder 2.

Vorteilhafte Ausführungsformen des erfindungsgemäßen Bauelementes ergeben sich aus den Maßnahmen in den abhängigen Ansprüchen.

Die gewählten Maßnahmen gewährleisten nunmehr, daß das erfindungsgemäße Bauelement zur Gaskonzentrationsmessung über bekannte automatisierte Bestückungsmethoden und -Anlagen auf Leiterplatten, Platinen oder dergleichen angeordnet werden kann. Es resultiert ein höherer Automatisierungsgrad bei der Fertigung von Systemen, die derartige Bauelemente enthalten. Beispielsweise lassen sich erfindungsgemäß ausgestaltete Bauelemente vorteilhaft im Bereich der KFZ-Elektronik zur Feuchtemessung einsetzen.

Aufgrund der Bauform des erfindungsgemäßen Bauelementes kann hierzu etwa vorgesehen werden, das entsprechende Bauelement mittels eines bekannten Vakuum-Manipulators zu ergreifen und auf einer Platine zu positionieren. Die elektrische Kontaktierung kann je nach gewünschtem Kontaktierungsprinzip geeignet gewählt werden, beispielsweise eine sog. pin-in-hole- Kontaktierung, eine übliche SMD-Kontaktierung etc..

Die erfindungsgemäßen Maßnahmen gewährleisten desweiteren einen äußerst kompakten und einfachen Aufbau des Bauelementes sowie eine entsprechend einfache Fertigung desselben. Gleichzeitig ist jedoch ein Schutz des im Bauelement angeordneten Sensorelementes vor einer mechanischen Beschädigung weitgehend gewährleistet, da dieses vollständig von geeigneten Materialien umkapselt ist. Die erfindungsgemäße Wahl dieser Materialien stellt zudem sicher, daß ein Gasaustauch in Richtung des Sensorelementes erfolgen kann.

Desweiteren ist sichergestellt, daß das eingesetzte jeweilige Sensorelement aufgrund der erfindungsgemäßen Ausgestaltung zuverlässig vor Verschmutzung geschützt ist.

Als weiterer Vorteil ist anzuführen, daß durch die geeignete Wahl des Filtermateriales sowie die Einstellung einer definierten Porengröße des Filtermateriales eine optimale Anpassung des Bauelementes an verschiedenste Applikationen möglich ist. Unter anderem kann derart etwa die Ansprechgeschwindigkeit des eingesetzten Sensorelementes gezielt eingestellt werden. Hierbei sei zudem darauf hingewiesen, daß selbstverständlich unlerschiedlichste Gase bzw. Gaskonzentrationen mittels des erfindungsgemäß ausgestalteten Bauelementes detektiert werden können.

Die erfindungsgemäßen Maßnahmen können selbstverständlich nicht nur bei Bauelementen zur Feuchtemessung in Gasen eingesetzt werden sondern bei verschiedenartigsten anderen Bauelementen zur Gaskonzentrationsmessung, wie etwa zur bereits oben erwähnten CO₂-Detektion in Luft, zur Detektion von Kohlenmonoxid CO. Schwefelmonoxid Soₓ, Stickoxiden Noₓ. usw..

Weitere Vorteile sowie Einzelheiten des erfindungsgemäßen Bauelementes ergeben sich aus der nachfolgenden Beschreibung mehrerer Ausführungsbeispiele anhand der beiliegenden Zeichnungen.

Dabei zeigt
- Figur 1a und 1b: je eine Teilansicht eines ersten Ausführungsbeispiels eines erfindungsgemäßen Bauelementes;
- Figur 2: eine seitliche Schnittansicht eines zweiten Ausführungsbeispiels eines erfindungsgemäßen Bauelementes in Verbindung mit einer Leiterpatte;
- Figur 3: eine perspektivische Teildarstellung eines dritten Ausführungsbeispieles eines erfindungsgemäßen Bauelementes;
- Figur 4a und 4b: je eine Ansicht eines kapazitiven Sensorelementes, wie es in den verschiedenen Ausführungsbeispielen des erfindungsgemäßen Bauelementes eingesetzt werden kann;
- Figur 5: eine seitliche Schnittansicht eines vierten Ausführungsbeispiels eines erfindungsgemäßen Bauelementes;
- Figur 6: eine seitliche Schnittansicht eines fünften Ausführungsbeispieles eines erfindungsgemäßen Bauelementes.

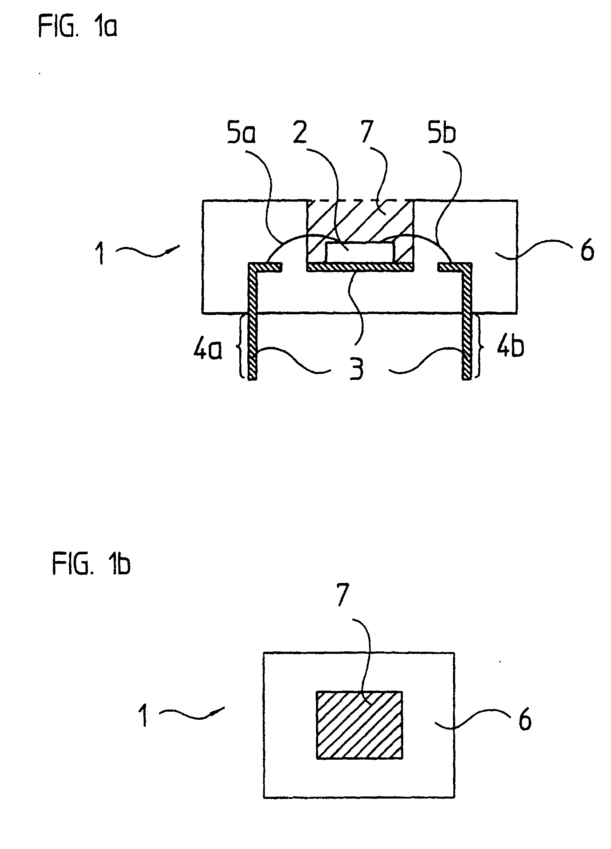

Ein erstes Ausführungsbeispiel eines erfindungsgemäßen Bauelementes 1 zur Gaskonzentrationsmessung ist in den Figuren 1a und 1b in schematisierten Darstellungen gezeigt, wobei diese Variante beispielsweise zur Feuchtemessung in Luft geeignet ist. Insbesondere anhand der seitlichen Schnitttdarstellung in Figur 1a sei nachfolgend der prinzipielle Aufbau des Bauelementes erläutert.
So umfaßt das dargestellte Ausführungsbeispiel des Bauelementes 1 ein feuchteempfindliches Sensorelement 2. welches in Abhängigkeit der Feuchte bzw. des Wasserdampfgehaltes des umgebenden Gases, in der Regel Luft, ein entsprechendes Ausgangssignal an eine nachgeordnete - nicht dargestellte - Auswerteeinheit liefert.

Im allgemeinen Fall ist das jeweilige Sensorelement entsprechend der gewünschten, zu detektierenden Gasart geeignet zu wählen, also etwa zur Wasserdampfdetektion, zur CO₂-Delektion etc..

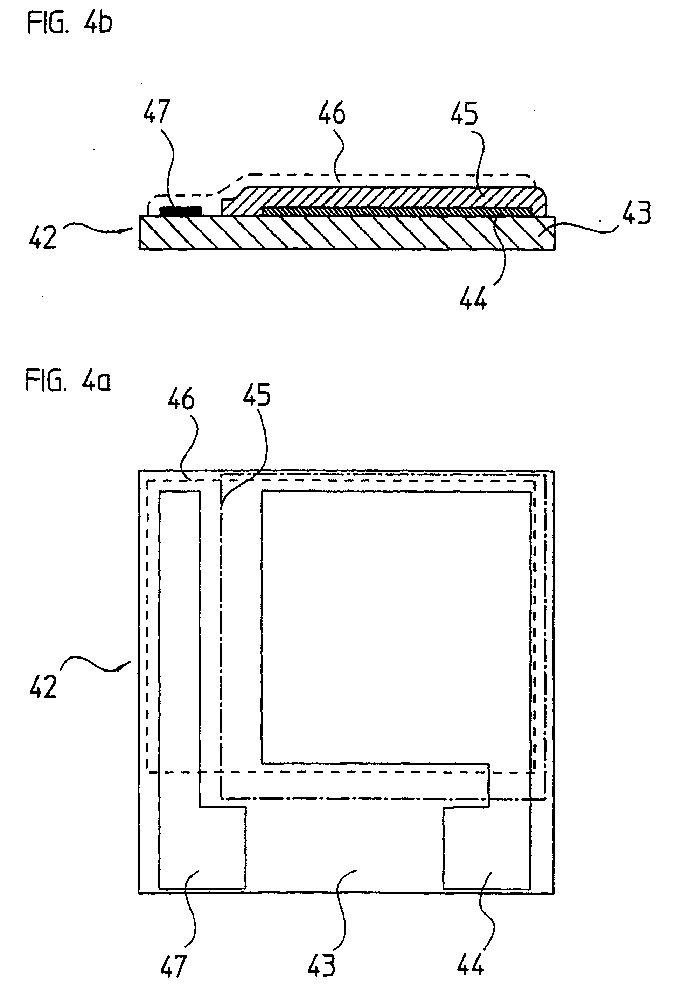

In Bezug auf eine mögliche Ausführung eines kapazitiven Sensorelementes zur Feuchtemessung 2 sei auf die nachfolgende Beschreibung der Figuren 4a und 4b verwiesen. Bereits an dieser Stelle sei jedoch darauf hingewiesen, daß selbstverständlich verschiedenste Varianten von Sensorelementen 2 im erfindungsgemäßen Bauelement 1 eingesetzt werden können.

Das Sensorelement 2 ist mittels eines geeigneten Klebstoffes auf einem Trägerelement 3 angeordnet, das zumindest teilweise elektrisch leitfähig ist und etwa als Metall-Stanzteil aus verzinntem Kupfer ausgebildet ist. Üblicherweise wird das Trägerelement 3 auch als sog. "Lead Frame" bezeichnet. Mit dem Trägerelement 3 ist das Sensorelement 2 ausgangsseitig über Bonddrähte 5a, 5b elektrisch leitend verbunden. Über Bereiche des Trägerelementes 3, die als Anschlußkontakte 4a, 4b dienen, kann das komplette Bauelement 1 mit einer geeigneten, nachgeordneten Auswerteeinheit elektrisch leitend verbunden werden. In der Auswerteinheit erfolgt die Weiterverarbeitung der vom Sensorelement 2 gelieferten - in diesem Fall feuchteabhängigen - Ausgangssignale.
Erfindungsgemäß ist in diesem Ausführungsbeispiel nunmehr vorgesehen. das Trägerelement 3 sowie das Sensorelement 2 mit einem ersten Material 6 zu umgeben, wobei die daraus resultierende räumliche Form des Bauelementes 1 so gewählt ist. daß letztlich ein Bauelement 1 resultiert, das eine automatisierte Bestückung von Leiterplatten oder Platinen ermöglicht. Hierzu weist das Bauelement 1 aufgrund der geeigneten Formgebung des ersten Materiales 6 im wesentlichen plane und glatte Grenzflächen auf: im gezeigten Beispiel resultiert ein quaderförmig ausgebildetes Bauelement 1 bzw. ein quaderförmiger Bauelement-Grundkörper. Als geeeigneles Material 6 kann beispielsweise eine bekannte Kunststoff-Vergußmasse. z.B. EpoxydHarz, verwendet werden, wie sie beispielsweise auch bei Standard-ICs verwendet wird.

Desweiteren ist erfindungsgemäß vorgesehen. zumindest in einem Fensterbereich des Bauelementes 1 ein zweites, anderes Material 7 anzuordnen. Während in diesem Ausführungsbeispiel das erste Material 6 undurchlässig für das umgebende Gas ist, ist das im Fensterbereich des Bauelementes 1 angeordnete zweite Material 7 als Filtermaterial ausgebildet, das teildurchlässig für das umgebende Gas ist. Derart ist gewährleistet, daß das umgebende Gas auf den empfindlichen Bereich des Sensorelementes 2 gelangen kann. Im ersten Ausführungsbeispiel befindet sich etwa der feuchteempfindliche Bereich des Sensorelementes 2 auf der Oberseite desselben.
Als geeignetes zweites Material 7, d.h. als Filtermaterial, kommt beispelsweise Polytetrafluoräthylen (PTFE), Polyäthlen (PE) oder aber deren Copolymere in Betracht. Alternativ hierzu könnten als Filtermaterial ein Sintermaterial eingesetzt werden, das in einem Sinterprozess hergestellt wird, wobei im Sinterprozess eine definierte Porengröße und damit eine definierte Durchlässigkeit des Filtermateriales einstellbar ist. Selbstverständlich kann erfindungsgemäß auch PTFE oder aber PE in gesinterter Form hierzu eingesetzt werden.

Ein derart ausgebildetes, erfindungsgemäßes Bauelement 1 zur Gaskonzentrationsmessung kann nunmehr aufgrund der räumlichen Bauform über bekannte Bestückungsverfahren automatisiert auf Leiterplatten, Platinen etc. angeordnet werden. Die elektrisch leitende Verbindung zwischen dem Bauelement 1 mit den auf der Leiterplatte vorgesehenen elektrischen Verbindungsleitungen bzw. Leiterbahnen und ggf. Signalverarbeitungs-Komponenten erfolgt über die als Anschlußkontakte 4a, 4b dienenden Bereiche des Trägerelementes 3, die aus dem Bauelement-Grundkörper herausragen.

Selbstverständlich existieren verschiedene weitere Möglichkeiten bezüglich der resultierenden Geometrie des erfindungsgemäßen Bauelementes 1. insbesondere bezüglich des Bauelement-Grundkörpers. Es muß bei der Formgebung der Materialien 6 und 7. die das Trägerelement 3 und das Sensorelement 2 umgeben, lediglich gewährleistet sein. daß in etwa plane und glatte Begrenzungsflächen resultieren, so daß über bekannte Bestückungsverfahren eine automatisierte Verarbeitung des Bauelementes 1, insbesondere ein automatisiertes Handling desselben möglich ist. Denkbar wäre desweiteren etwa auch eine würfelförmige Form des Bauelement-Grundkörpers etc..

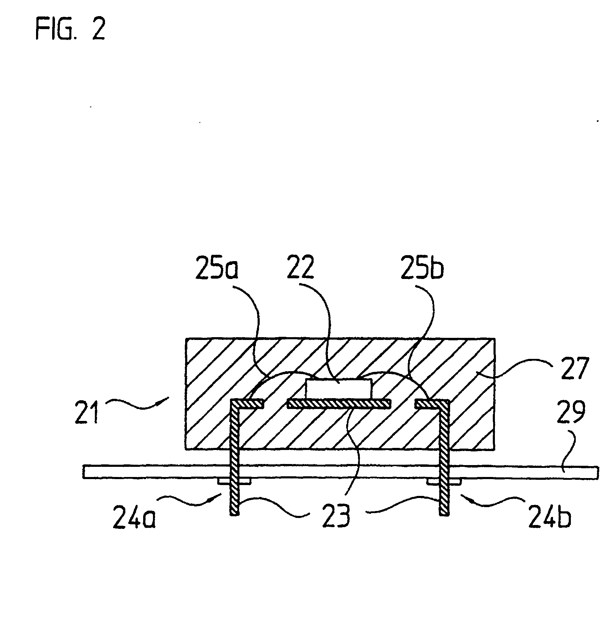

Ein zweites Ausführungsbeispiel eines erfindungsgemäßen Bauelementes 21 ist in Figur 2 schematisiert in einer Schnittdarstellung gezeigt. Das Bauelement 21 ist dabei auf einer Leiterplatte 29 angeordnet, die z.B. weitere - nicht dargestellte - signalverarbeitende Komponenten tragen kann. Wiederum umfaßt das erfindungsgemäße Bauelement 21 ein Trägerelement 23, auf dem erneut ein feuchteempfindliches Sensorelement 22 angeordnet ist. Eine elektrisch leitfähige Verbindung zwischen dem Trägerelement 23 und dem Sensorelement 22 ist über die Bonddrähte 25a, 25b sichergestellt. Die nach unten ragenden Teilbereiche 24a, 24b des Trägerelementes 23, welche aus dem Bauelement-Grundkörper herausragen, dienen zur Kontaktierung des Bauelementes 21 auf der Leiterplatte 29 bzw. zur elektrisch leitfähigen Verbindung des Bauelementes 21 mit den Leiterbahnen auf der Leiterplatte 29.
Im Unterschied zum ersten Ausführungsbeispiel ist nunmehr jedoch vorgesehen, das Sensorelement 22 sowie den größten Teil des Trägerelementes 23 mit einem einzigen Material 27 zu umgeben und durch eine entsprechende räumliche Formgebung desselben die automatisierte Verarbeitung des Bauelementes 21 zu ermöglichen. Insgesamt resultiert im gezeigten Ausfühurngsbeispiel wiederum eine quaderförmige Bauform des Bauelementes 21 mit im wesentlichen planen Grenzflächen, so daß beispielsweise über bekannte Vakuum-Manipulatoren das Bauelement 21 auf einer Leiterplatte 29 geeignet positioniert werden kann.
Als einziges Material 27 zur weitgehenden Umkapselung von Sensorelement 22 und Trägerelement 23 wird nunmehr jedoch ein Filtermaterial gewählt. das durchlässig für das zu detektierende Gas, z.B. Wasserdampf, ist. Hierzu kommen wiederum die gleichen Materialien wie im oben beschriebenen, ersten Ausführungsbeispiel in Betracht.
Diese Ausführungsform des erfindungsgemäßen Bauelemnetes 21 ist insofern noch einfacher herstellbar als das Bauelement im oben beschriebenen ersten Ausführungsbeispiel, da hierbei nunmehr lediglich ein einziges Material 27 entsprechend formgebend bearbeitet werden muß. Ebenso ist jedoch wiederum sichergestellt, daß durch das durchlässige Filtermaterial 27 z.B. die umgebende Luft mit dem darin enthaltenen Wasserdampf in Kontakt mit dem empfindlichen Bereich des Sensorelementes 22 kommt.

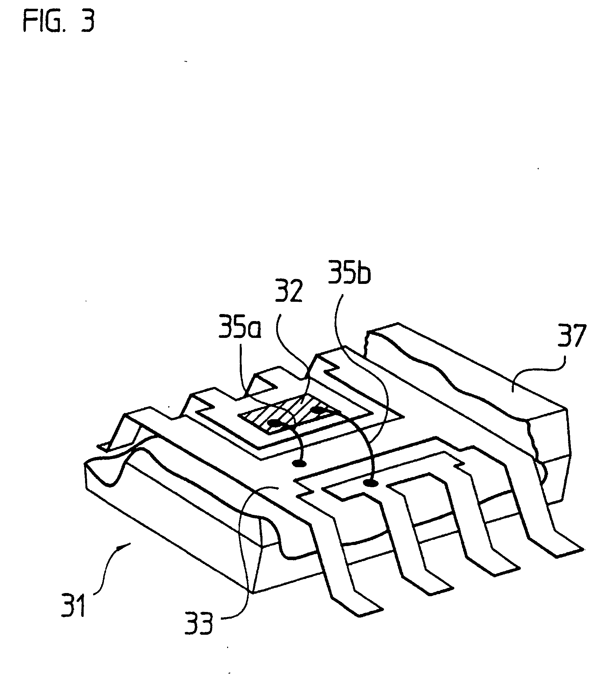

Eine dritte Ausführungsform eines erfindungsgemäß ausgebildeten Bauelementes 31 zur Gaskonzentrationsmessung ist in einer perspektivischen Teildarstellung in Figur 3 gezeigt.
Grundsätzlich entspricht dieses Ausführungsbeispiel demjenigen aus Figur 2. d.h. es ist vorgesehen, das Trägerelement 33 mit Ausnahme der Kontaktierungsbereiche sowie das Sensorelement 32 vollständig mit einem einzigen Material 37 zu umgeben bzw. zu umkapseln. Hierbei ist das umgebende Material 37, welches den Bauelement-Grundkörper bildet wiederum als geeignetes Filtermaterial ausgebildet, wie bereits oben erläutert wurde.
Deutlich erkennbar ist in der schematischen Darstellung der Figur 3 nunmehr, daß einzelne Teilbereiche des Trägerelementes 33 nunmehr als elektrische Anschlußkontakte genutzt werden, über die dem Sensorelement 32 geeignete Auswertekomponenten nachgeordnet werden können. Die elektrische leilfähige Verbindung zwischen diesen als Anschlußkontakten dienenden Teilbereichen und dem Sensorelement 32 erfolgt über Bonddrähte 35a, 35b. Einzelne Teilbereiche des Trägerelementes 33, die über den Bauelement-Grundkörper hinausragen, fungieren als Stützelemente lediglich zur Abstützung des Bauelementes 31, etwa bei der Anordnung auf einer Leiterplatte, und sind nicht zur Kontaktierung vorgesehen.
Ebenso soll über dieses Ausführungsbeispiel veranschaulicht werden, daß in Bezug auf die geometrische Ausgestaltung des Trägerelementes 33 selbstverständlich diverseste Möglichkeiten existieren.
Die gewählte räumliche Form des Bauelement-Grundkörpers mit im wesentlichen planen und glatten Grenzflächen ermöglicht wiederum die automatisierte Bestückung von Leiterplatten.

Ein geeignetes feuchteeempfindliches Sensorelement. wie es in den verschiedenen Ausführungsformen des erfindungemäßen Bauelementes eingesetzt werden kann, sei nachfolgend anhand der Figuren 4a und 4b kurz beschrieben. Ein derart aufgebautes kapazitives Sensorelement 42 zur Messung der Luftfeuchte ist im übrigen aus der WO 91/03735 der Anmelderin bekannt.
So besteht das Sensorelement 42 aus einem Trägersubstrat 43. vorzugsweise Glas, auf dem eine flächige Grundelektrode 44 aufgebracht ist. Ebenso ist auf dem Trägersubstrat 43 eine Anschlußelektrode 47 für die feuchtedurchlässige Deckelektrode 46 vorgesehen. Zwischen der Grundelektrode 44 und durchlässigen Deckelektrode 46 ist eine dünne Polymerschicht als Dielektrikum 45 angeordnet, deren Kapazität sich feuchteabhängig ändert.

Grundsätzlich kann selbstverständlich neben einem derartigen kapazitiven feuchteempfindlichen Sensorelement 42 auch ein bekanntes resistives Sensorelement im erfindungsgemäßen Bauelement zum Einsatz kommen.

Im Fall anderer, zu delektierender Gasarten, wie etwa bei der erwähnten CO₂-Detektion, ist selbstverständlich auch ein entsprechend modifiziertes Sensorelement einzusetzen.

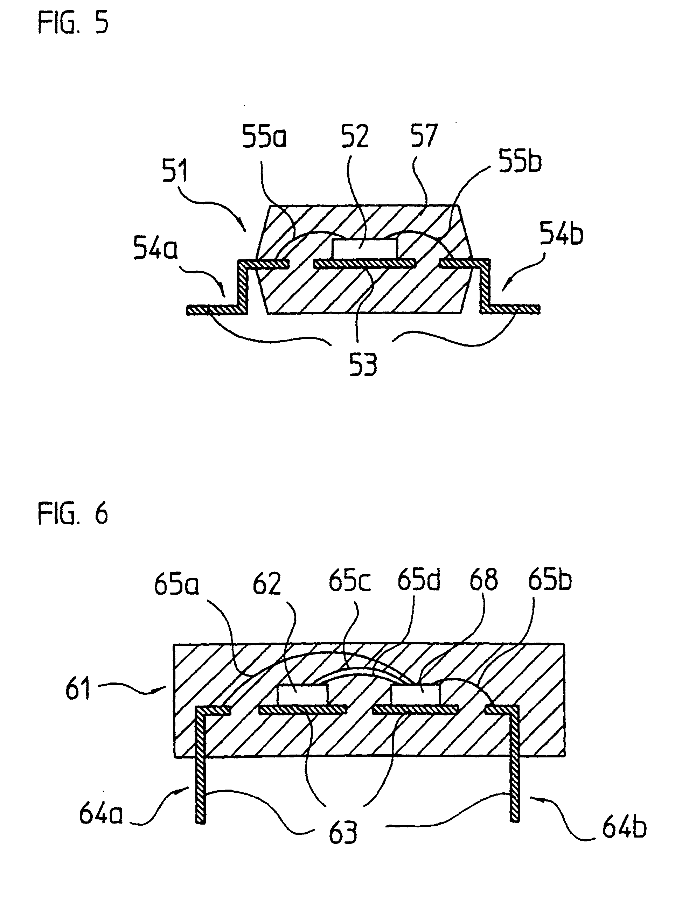

Zwei weitere mögliche Ausführungsformen des erfindungsgemäßen Bauelementes seien abschließend anhand der beiden Figuren 5 und 6 beschrieben.
Die in Figur 5 gezeigte Variante eines erfindungsgemäßen Bauelementes 51 entspricht bezüglich des grundsätzlichen Aufbaus dabei dem Ausführungsbeispiel aus Figur 2. Das heißt, das auf dem Trägerelement 53 angeordnete und über die Bonddrähte 55a, 55b damit verbundene Sensorelement 52 sind wiederum nahezu vollständig mit einem Filtermaterial 57 umgeben, das durchlässig für das umgebende Gas ist. Ferner ist das Filtermaterial 57 bezüglich der resultierenden Bauform des Bauelement-Grundkörpers so gestaltet, daß eine automatisierte Bestückung von Leiterplatten möglich ist.
Lediglich die Art und Weise der Kontaktierung des Bauelementes 51 mit der Leiterplatte ist in einer anderen Form vorgesehen, wozu die nach außen ragenden Anschlußkontakte 54a, 54b des Bauelementes 51 geeignet geometrisch diemensioniert sind. Während die Variante gemäß Figur 2 zur sog, pin-in-hole Bestückung geeignet ist, kann das Bauelement 51 gemäß Figur 5 über bekannte SMD-Bestückungsverfahren auf Leiterplatten angeordnet werden.
Selbstverständlich können die Anschlußkontakte des Bauelementes, die aus dem erfindungsgemäß ausgestalteten Bauelement-Grundkörper auch anders geometrisch dimensioniert werden, je nach der erforderlichen Kontaktierungsart auf der Leiterplatte oder Platine.

Ein fünftes Ausführungsbeispiel eines erfindungsgemäßen Bauelementes 61 sei abschließend anhand der Figur 6 erläutert. Vom grundsätzlichen Aufbau her entspricht auch diese Variante dem Ausführungsbeispiel der Figur 2. So ist auf einem Trägerelement 63 ein wiederum feuchteempfindliches Sensorelement 62 angeordnet. Desweiteren ist nunmehr jedoch vorgesehen, auf dem Trägerelement 63 ein Signalverarbeitungselement 68 anzuordnen, dem über geeignete Signalleitungen in Form von Bonddrähten 65c, 65d die Ausgangssignale des Sensorelementes 62 zugeführt werden. Im Signalverarbeitungselement 68 erfolgt eine Verarbeitung der vom Sensorelement 62 übergebenen Signale, d.h. eine Auswertung der Sensorsignale. Je nach gewünschtem Signalverarbeitungsgrad kann dieses Element 68 unterschiedlich ausgelegt werden, was bis hin zu einem kompletten ASIC gehen kann. Das Signalverarbeitungselement 68 ist über die Bonddrähte 65a, 65b mit den als Anschlußkontakten 64a, 64b dienenden Teilbereichen des Trägerelementes 63 verbunden.

Selbstverständlich existieren neben den erläuterten Varianten auch noch alternative Ausgestaltungsmöglichkeiten im Rahmen der vorliegenden Erfindung, welche durch die Ansprüche definiert wird.

## Patentansprüche

1. Bauelement zur Gaskonzentrationsmessung, bestehend aus einem Sensorelement (2; 42), das ein von der zu messenden Gaskonzentration abhängiges Sensorsignal liefert, und einem stabilen, zumindest teilweise leitfähigem Trägerelement (3), auf dem das Sensorelement (2) angeordnet und mit dem das Sensorelement (2) elektrisch leitend verbunden ist, wobei das Trägerelement (3) mehrere Anschlusskontakte (4a, 4b) aufweist, um das Sensorelement (2) mit einer Auswerteschaltung zu verbinden und das Trägerelement (3) sowie das Sensorelement (2) mit einem ersten Material (6) umgeben sind, wobei aus dem ersten Material (6) die Anschlusskontakte (4a, 4b) des Trägerelementes (3) herausragen und die räumliche Form des resultierenden Bauelementes derart gewählt ist, dass damit eine automatische Bestückung von Leiterplatten möglich ist; und desweiteren ein teildurchlässiges zweites Material (7) in einem Fensterbereich des ersten Materiales (6) angeordnet ist, so dass ein umgebendes Gas mit einem empfindlichen Bereich des Sensorelementes (2) in Kontakt kommt, und desweiteren das erste Material (6), das zweite Material (7), das Trägerelement (3) und das Sensorelement (2) formschlüssig zusammengefügt sind.

2. Bauelement zur Gaskonzentrationsmessung, bestehend aus einem Sensorelement (22; 32; 42; 52; 62), das ein von der zu messenden Gaskonzentration abhängiges Sensorsignal liefert, und einem stabilen, zumindest teilweise leitfähigem Trägerelement (23; 33; 53; 63), auf dem das Sensorelement (22; 32; 42; 52; 62) angeordnet und mit dem das Sensorelement (22; 32; 42; 52; 62) elektrisch leitend verbunden ist, wobei das Trägerelement (23; 33; 53; 63) mehrere Anschlusskontakte (24a, 24b; 54a, 54b; 64a, 64b) aufweist, um das Sensorelement (22; 32; 42; 52; 62) mit einer Auswerteschaltung zu verbinden und das Trägerelement (23; 33; 53; 63) sowie das Sensorelement (22; 32; 42; 52; 62) mit einem gasdurchlässigen einzigen Material (27; 37; 57) formschlüssig vollständig umkapselt sind, aus dem lediglich die Anschlusskontakte (24a, 24b; 54a, 54b; 64a, 64b) des Trägerelementes (23; 33; 53; 63) herausragen und die räumliche Form des resultierenden Bauelementes derart gewählt ist, dass damit eine automatische Bestückung von Leiterplatten möglich ist.

3. Bauelement nach Anspruch 1, wobei das erste Material (6) eine Kunststoff-Vergussmasse ist.

4. Bauelement nach Anspruch 1 oder 2, wobei das zweite Material (7) bzw. das gasdurchlässige einzige Material (27; 37; 57) ein gasdurchlässiges Filtermaterial ist.

5. Bauelement nach Anspruch 4, wobei das Filtermaterial (7; 27; 37; 57) Polytetrafluoräthylen (PTFE) ist.

6. Bauelement nach Anspruch 4, wobei das Filtermaterial (7; 27; 37; 57) Polyäthylen (PE) ist.

7. Bauelement nach Anspruch 4, wobei das Filtermaterial (7; 27; 37; 57) ein Sintermaterial ist, dessen Porengröße beim Herstellungsprozeß definiert einstellbar ist.

8. Bauelement nach Anspruch 5 oder 6, wobei das Filtermaterial (7; 27; 37; 57) als Copolymer von PE oder PTFE gewählt ist.

9. Bauelement nach Anspruch 1 oder 2, wobei das Trägerelement (3; 23; 33; 53; 63) neben den Anschlußkontakten (4a, 4b; 24a, 24b; 54a, 54b; 64a, 64b) auch Stützelemente aufweist, die aus dem Bauelement herausragen.

10. Bauelement nach Anspruch 1 oder 2, wobei das resultierende Bauelement im wesentlichen plane Grenzflächen aufweist.

11. Bauelement nach Anspruch 10, wobei das resultierende Bauelement im wesentlichen quader- oder würfelförmig ausgebildet ist.

12. Bauelement nach Anspruch 1 oder 2, wobei die herausragenden Anschlußkontakte (4a, 4b; 24a, 24b; 54a, 54b; 64a, 64b) des Trägerelementes (3; 23; 33; 53; 63) zur pin-in-hole Kontaktierung oder zur SMD-Bestückung des Bauelementes geeignet sind.

13. Bauelement nach Anspruch 1 oder 2, wobei das Sensorelement (42) als Feuchtesensor ausgebildet ist.

14. Bauelement nach Anspruch 13, wobei das Sensorelement (42) aus einem Glas-Trägersubstrat (43) besteht, auf dem eine erste flächige Grundelektrode (44) angeordnet ist, auf der wiederum ein Dielektrikum (45) angeordnet ist, welches feuchteabhängig seine Dielektrizitätskonstante verändert und auf dem Dielektrikum wiederum eine zweite flächige Deckelektrode (46) angeordnet ist und die beiden Elektroden mit je einem Anschlußkontakt des Trägerelementes leitend verbunden sind.

15. Bauelement nach Anspruch 1 oder 2, wobei innerhalb des Bauelementes desweiteren ein Signalverarbeitungselement (68) angeordnet ist, dem die Sensorsignale zugeführt werden, wobei das Signalverarbeitungselement (68) mit den Anschlußkontakten (64a, 64b) des Trägerelementes (63) leitend verbunden ist.

## Claims

1. Component for measuring gas concentration, comprising a sensor element (2; 42), which supplies a sensor signal dependent on the gas concentration to be measured, and a stable, at least partially conductive support element (3), on which the sensor element (2) is arranged and with which the sensor element (2) is connected in an electrically conductive manner, the support element (3) having a plurality of connecting contacts (4a, 4b) so as to connect the sensor element (2) with an evaluation circuit, and the support element (3) and the sensor element (2) being surrounded with a first material (6) from which the connecting contacts (4a, 4b) of the support element (3) protrude, and the physical form of the resulting component being so selected that automatic insertion of printed circuit boards is possible with it; and furthermore a partially permeable second material (7) is arranged in a window area of the first material (6) such that a surrounding gas comes into contact with a sensitive region of the sensor element (2), and furthermore the first material (6), the second material (7), the support element (3) and the sensor element (2) are joined together in an interlocking manner.

2. Component for measuring gas concentration, comprising a sensor element (22; 32; 42; 52; 62), which supplies a sensor signal dependent on the gas concentration to be measured, and a stable, at least partially conductive support element (23; 33; 53; 63) on which the sensor element (22; 32; 42; 52; 62) is arranged and with which the sensor element (22; 32; 42; 52; 62) is connected in an electrically conductive manner, the support element (23; 33; 53; 63) having a plurality of connecting contacts (24a, 24b; 54a, 54b; 64a, 64b) so as to connect the sensor element (22; 32; 42; 52; 62) with an evaluation circuit, and the support element (23; 33; 53; 63) and the sensor element (22; 32; 42; 52; 62) being completely encapsulated in an interlocking manner by a gas-permeable single material (27; 37; 57) from which only the connecting contacts (24a, 24b; 54a, 54b; 64a, 64b) of the support element (23; 33; 53; 63) protrude, and the physical form of the resulting component being selected such that automatic insertion of printed circuit boards is possible with it.

3. Component according to claim 1, wherein the first material (6) is a plastics filling compound.

4. Component according to claim 1 or 2, wherein the second material (7) or respectively the gas-permeable single material (27; 37; 57) is a gas-permeable filter material.

5. Component according to claim 4, wherein the filter material (7; 27; 37; 57) is polytetrafluoro-ethylene (PTFE).

6. Component according to claim 4, wherein the filter material (7; 27; 37; 57) is polyethylene (PE).

7. Component according to claim 4, wherein the filter material (7; 27; 37; 57) is a sintered material, the pore size of which can be adjusted in a defined manner during the manufacturing process.

8. Component according to claim 5 or 6, wherein the filter material (7; 27; 37; - 57) is selected as a copolymer of PE or FIFE.

9. Component according to claim 1 or 2, wherein the support element (3; 23; 33; 53; 63) has besides the connecting contacts (4a, 4b; 24a, 24b; 54a, 54b; 64a, 64b) also supporting members which protrude from the component.

10. Component according to claim 1 or 2, wherein the resulting component has substantially planar boundary surfaces.

11. Component according to claim 10, wherein the resulting component is configured substantially cuboid or cubic.

12. Component according to claim 1 or 2, wherein the protruding connecting contacts (4a, 4b; 24a, 24b; 54a, 54b; 64a, 64b) of the support element (3; 23; 33; 53; 63) are suitable for making pin-in-hole contact or for SMD-equipping of the component.

13. Component according to claim 1 or 2, wherein the sensor element (42) is configured as a humidity sensor.

14. Component according to claim 13, wherein the sensor element (42) comprises a glass support substrate (43) on which a first flat basic electrode (44) is disposed, on which in turn a dielectric (45) is arranged which alters its dielectric constant as a function of the humidity, and a second flat top electrode (46) is arranged in turn on the dielectric, and the two electrodes are each connected to a connecting contact of the support element in a conductive manner.

15. Component according to claim 1 or 2, wherein there is arranged furthermore inside the component a signal processing element (68), to which the sensor signals are supplied, the signal processing element (68) being connected in a conductive manner to the connecting contacts (64a, 64b) of the support element (63).

## Revendications

1. Composant pour la mesure de la concentration de gaz, comprenant un détecteur (2 ; 42) qui délivre un signal de détecteur dépendant de la concentration en gaz à mesurer et un élément support (3), stable, au moins partiellement conducteur, sur lequel le détecteur (2) est disposé et auquel le détecteur (2) est relié de manière électro-conductrice, l'élément support (3) comportant plusieurs contacts de connexion (4a, 4b) aux fins de connecter le détecteur (2) à un circuit de traitement et l'élément support (3) et le détecteur (2) étant entourés d'un premier matériau (6), les contacts de connexion (4a, 4b) de l'élément support (3) étant amenés à l'extérieur du premier matériau (6) et la forme dans l'espace du composant ainsi formé étant choisie telle qu'un garnissage automatique de plaquettes à circuit imprimé soit possible, un second matériau (7) partiellement perméable étant en outre disposé dans une fenêtre aménagée dans le premier matériau (6), de telle sorte qu'un gaz environnant soit en contact avec une zone sensible du détecteur (2) et en outre le premier matériau (6), le second matériau (7), l'élément support (3) et le détecteur (2) étant assemblés par complémentarité de formes.

2. Composant pour la mesure de la concentration de gaz, comprenant un détecteur (22 ; 32 ; 42 ; 52 ; 62) qui délivre un signal de détecteur dépendant de la concentration en gaz à mesurer et un élément support (23 ; 33 ; 53 ; 63), stable, au moins partiellement conducteur, sur lequel le détecteur (22 ; 32 ; 42 ; 52 ; 62) est disposé et auquel le détecteur (22 ; 32 ; 42 ; 52 ; 62) est relié de manière électro-conductrice, l'élément support (23 ; 33 ; 53 ; 63) comportant plusieurs contacts de connexion (24a, 24b ; 54a, 54b ; 64a, 64b) aux fins de connecter le détecteur (22 ; 32 ; 42 ; 52 ; 62) à un circuit de traitement, l'élément support (23 ; 33 ; 53 ; 63) et le détecteur (22 ; 32 ; 42 ; 52 ; 62) étant entièrement encapsulés avec complémentarité de formes dans un matériau (27 ; 37 ; 57) unique perméable au gaz, hors duquel font saillie uniquement les contacts de connexion (24a, 24b ; 54a, 54b ; 64a, 64b) de l'élément support (23 ; 33 ; 53 ; 63) et la forme dans l'espace du composant ainsi formé étant choisie telle qu'un garnissage automatique de plaquettes à circuit imprimé soit possible.

3. Composant selon- la revendication 1, dans lequel le premier matériau (6) est une masse coulable de matière plastique.

4. Composant selon la revendication 1 ou 2, dans lequel le second matériau (7) ou le matériau unique (27 ; 37 ; 57) perméable au gaz est un matériau filtrant perméable au gaz.

5. Composant selon la revendication 4, dans lequel le matériau filtrant (7 ; 27 ; 37 ; 57) perméable au gaz est du polytetrafluoréthylène (PTFE).

6. Composant selon la revendication 4, dans lequel le matériau filtrant (7 ; 27 ; 37 ; 57) perméable au gaz est du polyéthylène (PE).

7. Composant selon la revendication 4, dans lequel le matériau filtrant (7 ; 27 ; 37 ; 57) est un matériau fritté dont la dimension des pores peut être réglée de manière précise à la fabrication.

8. Composant selon la revendication 5 ou 6, dans lequel le matériau filtrant (7 ; 27 ; 37 ; 57) est un copolymère de PE ou de PTFE.

9. Composant selon la revendication 1 ou 2, dans lequel l'élément support (3 ; 23 ; 33 ; 53 ; 63), en plus des contacts de connexion (4a, 4b ; 24a, 24b ; 54a, 54b ; 64a, 64b) présente des éléments de soutien qui font saillie hors du composant.

10. Composant selon la revendication 1 ou 2, dans lequel le composant obtenu présente des surfaces extérieures essentiellement planes.

11. Composant selon la revendication 10, dans lequel le composant obtenu présente une forme essentiellement parallélépipédique ou cubique.

12. Composant selon la revendication 1 ou 2, dans lequel les contacts de connexion (4a, 4b ; 24a, 24b ; 54a, 54b ; 64a, 64b) saillants de l'élément support (3 ; 23 ; 33 ; 53 ; 63) sont adaptés pour une connexion de type broche-trou ou CMS du composant.

13. Composant selon la revendication 1 ou 2, dans lequel le détecteur (42) est agencé en détecteur d'humidité.

14. Composant selon la revendication 13, dans lequel le détecteur (42) est formé d'un substrat-support en verre (43) sur lequel est disposée une première électrode de base (44) plane, sur laquelle à son tour est appliqué un diélectrique (45), dont la constante diélectrique varie en fonction de l'humidité, et une seconde électrode (46) de couverture est disposée sur le diélectrique; et les deux électrodes sont reliées de manière électro-conductrice chacune à un contact de connexion de l'élément support.

15. Composant selon la revendication 1 ou 2, dans lequel un élément de traitement de signal (68) auquel sont envoyés les signaux de détecteur est disposé à l'intérieur du composant, l'élément de traitement de signal (68) étant relié de manière électro-conductrice aux contacts de connexion (64a, 64b) de l'élément support (63).
